(19) Europäisches Patentamt
European Patent Office
Office européen des brevets

(11) EP 4 442 327 A1

(12) EUROPEAN PATENT APPLICATION

(43) Date of publication:
**09.10.2024 Bulletin 2024/41**

(21) Application number: **23382330.1**

(22) Date of filing: **05.04.2023**

(51) International Patent Classification (IPC):
***A61Q 19/10*** *(2006.01)* ***A61Q 5/02*** *(2006.01)*
***C11D 1/825*** *(2006.01)* ***C07C 1/00*** *(2006.01)*
***C07C 67/03*** *(2006.01)* ***C07C 67/26*** *(2006.01)*
***C07C 69/58*** *(2006.01)* ***C08G 65/26*** *(2006.01)*
***C11D 11/00*** *(2006.01)* ***C11D 11/04*** *(2006.01)*
***A61K 8/39*** *(2006.01)*

(52) Cooperative Patent Classification (CPC):
**C11D 1/825; A61K 8/39; A61Q 5/02; A61Q 19/10; C08G 65/2615; C11D 11/0094; C11D 11/04;**
A61K 2800/596; C11D 1/72; C11D 1/74

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **KAO CORPORATION, S.A.**
**08210 Barberà del Vallès,**
**Barcelona (ES)**

(72) Inventors:
- **CABRERA CRESPO, José**
  **E-08210 Barberà del Vallès, Barcelona (ES)**
- **PEY GUTIÉRREZ, Carmen Maria**
  **E-08210 Barberà del Vallès, Barcelona (ES)**
- **PI BOLEDA, Bernat**
  **E-08210 Barberà del Vallès, Barcelona (ES)**
- **NOGUÉS LÓPEZ, Blanca**
  **E-08210 Barberà del Vallès, Barcelona (ES)**

(74) Representative: **ABG Intellectual Property Law, S.L.**
**Avenida de Burgos, 16D**
**Edificio Euromor**
**28036 Madrid (ES)**

(54) **COMPOSITION WHICH CONTAINS A MIXTURE OF MONO-, DI-, AND TRIGLYCERIDES AND GLYCERINE**

(57) The present invention relates to a composition comprising a mixture of alkoxylated mono-, di-, and triglycerides and glycerine, to a method for the preparation of said composition, the detergent compositions which contain said composition and to the use of said composition as surfactant or co-surfactant in laundry detergent compositions, fabric care compositions, hard surface compositions, skin cleansing compositions, hair cleansing compositions and skin care compositions.

EP 4 442 327 A1

## Description

### FIELD OF THE INVENTION

The present invention relates to a composition comprising a mixture of alkoxylated mono-, di-, and triglycerides and glycerine, to a method for the preparation of said composition, the detergent compositions which contain said composition and to the use of said composition as surfactant or co-surfactant in laundry detergent compositions, fabric care compositions, hard surface compositions, skin cleansing compositions and hair cleansing compositions.

### STATE OF THE ART

Ethoxylated glycerol fatty acid esters are non-ionic surfactants comprising a mixture of ethoxylated mono-, di-, and triglycerides as well as glycerine. They are known to be mild surfactants with low eco-toxicological profile. They are used as a multifunctional tool in formulations, being used, for instance, as surfactant or co-surfactant, as cleaner, emulsifier, emollient or hydrotrope. Therefore they are used in laundry and household application as well as in cosmetic formulations.

There is also a concern in the consumer products market to formulate products with ingredients that are based on sources derived from plants or animals, rather than fossil fuels, in particularly derived from a vegetal origin. Such ingredients are considered "green" or "natural" since they are derived from renewable and/or sustainable sources. It is also of concern the use of vegetal raw materials obtained in tropical areas, due to deforestation and human rights abuse associated to the cultivation of those tropical oils, as well as the need of transportation for long distances of such raw materials to the main customer markets in Europe and North America, increasing the content of $CO_2$ released into the atmosphere. Thus there is a trend in using raw materials of vegetal origin obtained nearby, for instance, within Europe.

Ethoxylated glycerol fatty acid esters are used in different applications due to their relevant properties. For instance they are used in laundry and other cleansing applications such as laundry detergents, fabric care agents in laundry detergents, (manual) dishwashing formulations and hard surface cleaners, due to its thickening ability, detergency fulfillment, hydrotopy in liquid tablets, fabric care properties of laundry detergents such as softness or wrinkle reduction, soil removal performance as well as foam requirements (foam volume, foam duration, foam quantity, associated to a good cleaning effect, foam creaminess, associated to conditioning effect, foam density, foam texture, speed of foaming obtention and foam tolerance to hard water and presence of oil and/or greases). Ethoxylated glycerol fatty acid esters are also of special interest in cosmetic applications such as hair and skin cleansing application, due to its characteristics in thickening ability, foam requirements (foam speed, volume, creaminess), detangling and combability, rinsability and smoothness of hair and skin, as well as spreadability and absorbency.

Compositions comprising a mixture of alkoxylated mono-, di-, and triglycerides and glycerine are well known by persons skilled in the art.

EP0586323 describes a detergent composition with improved properties with respect to ecotoxicity and irritation to the eyes and skin; said composition comprises the mono-, di- and triester compounds represented by formula (I), wherein the proportion by weight of mono-, di-, and triester is 46-90/9-30/1-15

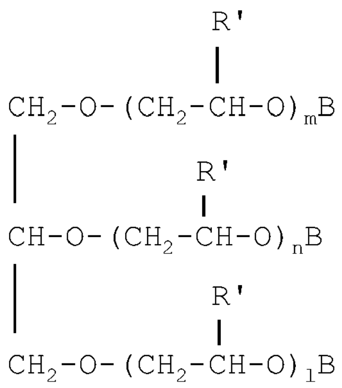

(I)

wherein R' represents H or $CH_3$, B represents H or the group represented by -CO-R, wherein R represents an alkyl or alkenyl group with C6-C22, and each one of n, m and 1 independently represent a whole number between 0 and 40; with n+m+l= 2-100, preferably 9-19,

and the compound represented by formula (II)

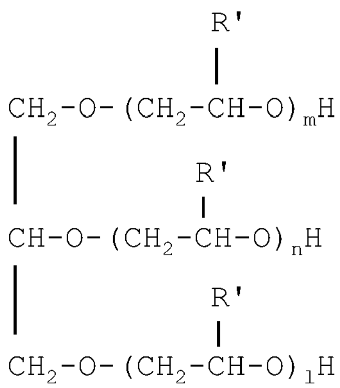

(II)

wherein each one of n, m and 1 independently represent a whole number between 0 and 40; with n+m+l= 2-100, preferably 9-19,wherein the proportion by weight (I)/(II) has a value between 3 and 0.33 (3:1 and 1:3), preferably between 1.3 and 0.75 (3:2.3 and 2.25:3)

EP1045021 describes a composition with high viscosity and good foam stability, as well as good properties with respect to biodegradability and irritation to the eyes and skin, said composition comprises

(i) components represented by formula (III), wherein, independently, each one of the symbols B1, B2 and B3 represents an acyl group represented by -CO-R
(ii) components represented by formula (III), wherein, independently, two of the symbols B1, B2 and B3 represent an acyl group represented by -CO-R and the remaining ones represent H;
(iii) components represented by formula (III), wherein, independently, one of the symbols B1, B2, B3 represents an acyl group represented by -CO-R and the remaining ones represent H;

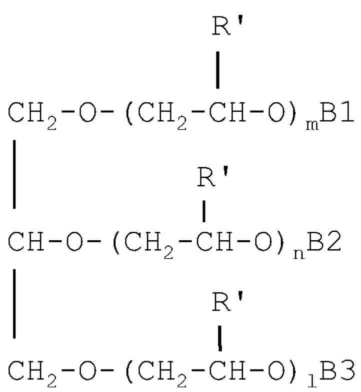

(III)

(iv) components represented by formula (III), wherein each one of B1, B2 and B3 represents H;

wherein the proportion by weight of components (iii)/(ii)/(i) is 46-90/9-35/1-15, preferably 60-83/16-35/1-6, wherein R' represents H or $CH_3$, and each one of m, n, or l represents, independently, a number from 0 to 4, the sum of m, n and l being in the range of 1 to 4, preferably in the range of 1 to a number less than 2, even more preferably from 1.5 to a number lower than 2, and in the acyl group represented by -CO-R, R represents an alkyl or alkenyl group of 6 to 22 carbon atoms, the proportion by weight (iii)+(ii)+(i)/(iv) being in the range of 85/15 to 40/60 (5.67:1 to 1:1.5), preferably from 80/20 to 45/55 (4:1 to 1:1.2).

EP2029711 describes a detergent composition comprising a mixture of mono-, di- and triester compounds represented by formula (I) as described above, and glycerine, wherein the proportion by weight of mono-, di-, and triester is 46-90/9-30/1-15, and wherein the hydrocarbon chain from the acyl group is from 6 to 11 carbon atoms. Patent describes dishwashing compositions with good foam requirements fulfilled.

EP1106675 describes the use of ethoxylated partial glyceride products which consist of mixtures of ethoxylated partial glycerides of formula (IV)

$CH_2$-O-CO-$R_1$
|
CH-O-$R_2$ (IV)
|
$CH_2$-O-$R_3$

wherein -CO-$R_1$ represents an acyl group of 6 to 22 carbon atoms, linear or branched, saturated or unsaturated, and $R_2$ and $R_3$ represent, independently from one another, hydrogen or -CO-$R_1$ with the condition that either $R_2$ or $R_3$ is hydrogen,
and ethoxylated glycerol in a proportion by weight in the range of 6:1 to 3.3:1 for the production of detergents for washing fabrics, detergents for dishwashing and cleaners, especially in the form of detergent tablets. According to, the proportion by weight of mono-, di-, and triester is 46-90/9-30/1-15, and the products which are used (mixtures of ethoxylated partial glycerides of formula IV) and ethoxylated glycerol) are addition products of 1 to 50 moles of ethylene oxide per mole of partial glyceride, preferably from 5 to 20 moles of ethylene oxide.

From the state of the art set forth above, it can be seen that there is still a need for compositions comprising a mixture of mono-, di-, triglycerides and glycerine that meet the requirements of compositions for cleansing and/or care of textiles or fabrics, dishware and hard surfaces cleaning, as well as for cosmetic cleansing compositions for skin and hair.

The present inventions aims at addressing the above-described needs, and provides a composition comprising a mixture of mono-, di-, triglycerides and glycerine, wherein they are obtained totally or partially from renewable and/or sustainable sources, such as raw materials of vegetal origin; a process of preparation of said composition; and its use in laundry detergency and cleaning of dishware and hard surfaces, as well as cosmetic applications. The compositions are able to comply with the technical and economic requirements imposed on them and provide good performance.

## SUMMARY OF THE INVENTION

The first aspect of the present invention is a composition comprising:

(i) at least one component represented by formula (I), wherein, independently, one of the symbols $B_1$, $B_2$, $B_3$ represents an acyl group represented by -CO-R and the remaining ones represent H;
(ii) at least one component represented by formula (I), wherein, independently, two of the symbols $B_1$, $B_2$ and $B_3$ represent an acyl group represented by -CO-R and the remaining one represents H;
(iii) at least one component represented by formula (I), wherein, independently, each one of the symbols $B_1$, $B_2$ and $B_3$ represents an acyl group represented by -CO-R;

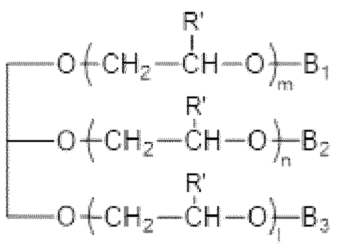

(I)

; and

(iv) at least one component represented by formula (II);

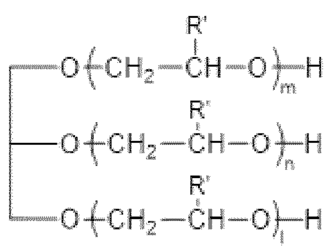

(II)

wherein each one of m, n, and l represents, independently, a number from 0 to 40,
the sum of m, n and l for each component (i)-(iv) is independently from 5 to 40, preferably from 5 to 30;
R' represents independently H or $CH_3$, preferably H;
wherein in the acyl group represented by -CO-R, each R represents independently a linear alkyl group or a monounsaturated or polyunsaturated linear alkenyl group, containing 6 to 22 carbon atoms, preferably 10 to 20 carbon atoms, more preferably 12 to 18 carbon atoms, even more preferably from 14 to 18 carbon atoms;
provided that at least one R group is a group of formula $R_1$ that is a monounsaturated or polyunsaturated linear alkenyl group having 18 carbon atoms;
wherein at least 50% by weight of the acyl groups -CO-R comprised in components (i), (ii) and (iii) of the composition are a -CO-$R_1$ group; and
wherein the ratio by weight of components (i) / (ii) / (iii) is of 5-90 / 10-50 / up to 65, preferably 15-85 / 15-50 / up to 45, more preferably 30-85 / 15-50 / up to 25;
and wherein the ratio by weight of the sum of components (i)+(ii)+(iii) to component (iv) is from 1:3 to 1:0.002, preferably from 1:2 to 1: 0.01, more preferably from 1:1.5 to 1:0.05.

The processes for preparing said composition also form part of the object of the invention.

Thus, the second aspect is a process for producing the composition according to the first aspect comprising the steps:

a) reacting glycerine with a component of formula (IIIa) in an interesterification reaction, wherein R is as defined in the first aspect;

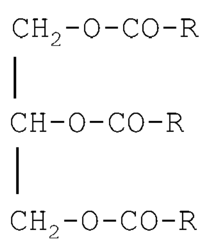

Formula (IIIa)

b) subjecting the reaction mixture obtained in step a) to an alkoxylation process using an alkylene oxide with 2 or 3 carbon atoms in the presence of an alkaline catalyst.

The third aspect is a process for producing the composition according to the first aspect, comprising the steps:

a') reacting glycerine with an alkylene oxide of 2 or 3 carbon atoms in the presence of an alkaline catalyst;
b') reacting the reaction mixture obtained in step a') with a component of formula (3)

R-COOX (3)

wherein R is as defined in the first aspect, and X represents a methyl group or H.

The fourth aspect is a process for producing the composition according to the first aspect, comprising the steps:

a") reacting glycerine with an alkylene oxide of 2 or 3 carbon atoms in the presence of an alkaline catalyst;
b") reacting the reaction mixture obtained in step a") with a component of formula (IIIa) as defined in the second aspect, wherein R is as defined in the first aspect.

The fifth aspect of the present invention is a detergent composition comprising a composition according to the first aspect or a composition obtainable by the process defined in the second, third or fourth aspects.

The sixth aspect of the invention is a process for producing the detergent composition according to the fifth aspect comprising the step of dispersing in water a composition as defined in the first aspect or obtainable by the process as defined the second, third or fourth aspects, preferably at a temperature in the range of 15 to 35 °C.

The seventh aspect of the invention is the use of the detergent compositions according to the fifth aspect for the cleansing and/or care of textiles or fabrics, dishware and hard surfaces as well as skin and hair.

The eighth aspect of the invention is a method to cleanse and/or care of textiles or fabrics, dishware and hard surfaces, as well as skin and hair, comprising the step of applying a detergent composition as defined in the fifth aspect

to textiles or fabrics, dishware and hard surfaces, as well as skin and hair.

A further aspect relates to the use of a composition according to the first aspect or a composition obtainable by the process as defined in the second, third or fourth aspects as surfactant or co-surfactant in a detergent composition, preferably wherein said detergent composition is selected from the group consisting of laundry detergent compositions, fabric care compositions, dishwashing compositions, hard surface detergent compositions or personal care compositions such as skin cleansing compositions, hair cleansing compositions and skin care compositions.

**DETAILED DESCRIPTION OF THE INVENTION**

MIXTURE OF MONO-, DI-, AND TRIGLYCERIDES AND GLYCERINE

The composition of the present invention comprises a mixture of alkoxylated mono-, di-, and triglycerides and glycerine, which is a composition comprising:

(i) at least one component represented by formula (I), wherein one of the symbols $B_1$, $B_2$, $B_3$ independently represents an acyl group represented by -CO-R and the remaining ones represent H;
(ii) at least one component represented by formula (I), wherein two of the symbols $B_1$, $B_2$ and $B_3$ independently represent an acyl group represented by -CO-R and the remaining one represents H;
(iii) at least one component represented by formula (I), wherein, each one of the symbols $B_1$, $B_2$ and $B_3$ independently represent an acyl group represented by -COR;

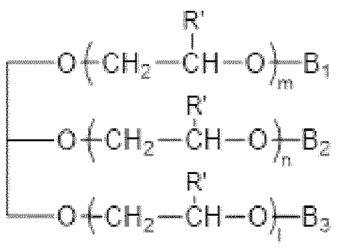

(I)

; and

(iv) at least one component represented by formula (II);

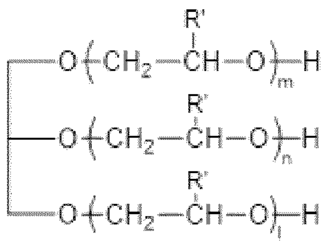

(II)

wherein each of m, n and l independently represents a number from 0 to 40,
the sum of m, n, l for each component (i)-(iv) is independently from 5 to 40, preferably from 5 to 30;
R' represents independently H or $CH_3$, preferably H;
wherein in the acyl groups represented by -CO-R, each R represents independently a linear alkyl group or a monounsaturated or polyunsaturated linear alkenyl group, containing 6 to 22 carbon atoms, preferably 10 to 20 carbon atoms, more preferably 12 to 18 carbon atoms, even more preferably from 14 to 18 carbon atoms; provided that at least one R group is a group of formula $R_1$ that is a monounsaturated or polyunsaturated linear alkenyl group having 18 carbon atoms;
wherein at least 50% by weight of the acyl groups -CO-R comprised in components (i), (ii) and (iii) of the composition are a -CO-$R_1$ group;
wherein the ratio by weight of components (i)/(ii)/(iii) is 5-90 / 10-50 / up to 65, preferably from 15-85 / 15-50 / up to 45, more preferably from 30-85 / 15-50 / up to 25; and wherein the ratio by weight of the sum of components (i)+(ii)+(iii) to component (iv), that is, (i)+(ii)+(iii)/(iv) is from 1:3 to 1:0.002, preferably from 1:2 to 1: 0.01, more preferably from 1:1.5 to 1:0.05.

Component (i) is an alkoxylated (ethoxylated or propoxylated, preferably ethoxylated) monoglyceride of the

compound of formula (I) since only one of the symbols $B_1$, $B_2$, $B_3$ independently represents an acyl group represented by -CO-R and the remaining ones represent H. Component (i) comprises at least one monoglyceride of the compound of formula (I), that means that component (i) may comprise a single monoglyceride of the compound of formula (I) or it may comprise a mixture of two or more monoglycerides of the compound of formula (I).

Component (ii) is an alkoxylated (ethoxylated or propoxylated, preferably ethoxylated) diglyceride of the compound of formula (I) since two of the symbols $B_1$, $B_2$, $B_3$ independently represents an acyl group represented by -CO-R and the remaining one represents H. Component (ii) comprises at least one diglyceride of the compound of formula (I), that means that component (ii) may comprise a single diglyceride of the compound of formula (I) or it may comprise a mixture of two or more diglycerides of the compound of formula (I).

Component (iii) is an alkoxylated (ethoxylated or propoxylated, preferably ethoxylated) triglyceride of the compound of formula (I) since each one of the symbols $B_1$, $B_2$ and $B_3$ independently represent an acyl group represented by -CO-R. Component (iii) comprises at least one triglyceride of the compound of formula (I), that means that component (iii) may comprise a single triglyceride of the compound of formula (I) or it may comprise a mixture of two or more triglycerides of the compound of formula (I) .

Component (iv) is an alkoxylated (ethoxylated or propoxylated, preferably ethoxylated) glycerine. Component (iv) comprises at least one alkoxylated glycerine of formula (II), that means that component (iv) may comprise a single alkoxylated glycerine of formula (II) or it may comprise a mixture of two or more alkoxylated glycerines of formula (II).

The compositions of the invention must comprise at least one component (i), at least one component (ii), at least one component (iii) and at least one component (iv).

In the compositions of the present invention, m, n and l are independently a number from 0 to 40, preferably from 0 to 20. In the compositions of the present invention, m, n and l have the same or different value, which is a number from 0 to 40, preferably from 0 to 20. In the compositions of the present invention, m is a number from 0 to 40, preferably from 0 to 20. In the compositions of the present invention, n is a number from 0 to 40, preferably from 0 to 20. In the compositions of the present invention, l is a number from 0 to 40, preferably from 0 to 20. Each component (i), (ii), (iii) and (iv) can have different values of m, n and l; components (i), (ii), (iii) and (iv) can have different values of m; components (i), (ii), (iii) and (iv) can have different values of n; components (i), (ii), (iii) and (iv) can have different values of l; components (i), (ii), (iii) and (iv) can have the same value of m; components (i), (ii), (iii) and (iv) can have the same value of n; and/or components (i), (ii), (iii) and (iv) can have the same values of l; said values being as defined herein. If the composition comprises more than one compound of formula (I) or (II) in components (i), (ii), (iii) and/or (iv), respectively, for each compound of formula (I) and (II) the m, n and l values falls within the ranges defined herein. If the composition comprises more than one compound of formula (I) or (II) in components (i), (ii), (iii) and/or (iv), respectively, the resulting average m, n and l values for each component (i), (ii), (iii) and/or (iv), also falls within the ranges defined herein.

In the compositions of the present invention, the sum of m, n and l for each component (i)-(iv) is independently from 5 to 40, preferably from 5 to 30; more preferably from 5 to 20, even more preferably from 5 to 15. The sum of m, n and l independently refers to the sum of m, n and l for each of the components (i), (ii), (iii), (iv) in the composition. The sum of m, n and l indicates the alkoxylation degree of each component of the composition. Each component of the composition can have the same or different alkoxylation degree. If the composition comprises more than one compound of formula (I) or (II) in components (i), (ii), (iii) and/or (iv), respectively, for each compound of formula (I) and (II) the sum of m, n and l values falls within the ranges defined herein. If the composition comprises more than one compound of formula (I) or (II) in components (i), (ii), (iii) and/or (iv), respectively, the resulting average sum of m, n and l values for each component (i), (ii), (iii) and/or (iv), also falls within the ranges defined herein.

In the compositions of the present invention, R' represents independently H or $CH_3$. When R' is H, the components are ethoxylated. When R' is $CH_3$ the components are propoxylated. Each components (i)-(iv) can have the a different value of R', even within a single component (i), (ii), (iii) or (iv), leading to a mixture of ethoxylated and propoxylated components. Each components (i)-(iv) can have the same value of R'. In a preferred embodiment R' is H in all components (i)-(iv).

In the compositions of the present invention, in the acyl groups represented by -CO-R, each R represents independently a linear alkyl group or a monounsaturated or polyunsaturated linear alkenyl group, containing 6 to 22 carbon atoms, preferably 10 to 20 carbon atoms, more preferably 12 to 18 carbon atoms, even more preferably 14 to 18 carbon atoms; provided that at least one R group in the composition is a group of formula $R_1$ that is a monounsaturated or polyunsaturated linear alkenyl group having 18 carbon atoms. The proviso of at least one R group in the composition being a group of formula $R_1$ that is a monounsaturated or polyunsaturated linear alkenyl group having 18 carbon atoms means that at least one of components (i) to (iv) has at least one R group that is $R_1$. In a preferred embodiment, each of components (i) to (iv) have at least one R group that is a monounsaturated or polyunsaturated linear alkenyl group having 18 carbon atoms.

As used herein, the term "linear alkyl" refers to a straight hydrocarbon chain containing the indicated number of carbon atoms, such as 6 to 22 carbon atoms, preferably 10 to 20 carbon atoms, more preferably 12 to 18 carbon atoms, more preferably from 14 to 20 carbon atoms, even more preferably from 14 to 18 carbon atoms.

As used herein, the term "monounsaturated or polyunsaturated linear alkenyl" refers to a linear hydrocarbon chain containing the indicated number of carbon atoms, such as 6 to 22 carbon atoms, preferably 10 to 20 carbon atoms, more preferably 12 to 18 carbon atoms, more preferably from 12 to 18, even more preferably from 14 to 18 carbon atoms and one or more unsaturations which are double bonds, preferably from 1 to 3 double bonds.

The term "monounsaturated" when referring to an "alkenyl" means that there is only one double bond.

The term "polyunsaturated" when referring to an "alkenyl" means that there are more than one double bonds, preferably from 2 to 3 double bonds.

In particular, the linear alkyl and monounsaturated or polyunsaturated linear alkenyl are unsubstituted, i.e. they do not have any substituent.

Linear alkyl or monounsaturated or polyunsaturated linear alkenyl hydrocarbon groups R can originate from fatty acids, or methyl esters/triglycerides thereof, or can be an alkyl or alkenyl derived from oils and fats from vegetal origin, such as palm, rapeseed, sunflower, soybean, olive, canola. Fatty acids, or methyl esters/triglycerides thereof, such as palmitoleic acid, oleic acid, elaidinic acid, petroselinic acid, linoleic acid, linolenic acid, stearic acid, myristic acid, and gadoleic acid or mixtures thereof, can also be employed in the present invention.

Linear or branched alkyl or linear alkenyl hydrocarbon group R can originate from fatty acids, or methyl esters/triglycerides thereof, or can be an alkyl or alkenyl derived from vegetable oils, vegetable waxes, vegetable fats or vegetable resins selected from the group comprising amaranth, anise, apple, apricot, arnica, avocado, cotton, borage, broccoli, hemp, hazelnut, beech, boxwood, thistle, spelt, peanut, yellow nutsedge, lilac, garden cress, barley, pomegranate, oats, vaccinium, elder, jasmine, jatropha, currant, St. John's wort, jojoba, camellia, chamomile, caraway, carrot, cherry, coriander, mullein, crambe, caper spurge, squash, dragon head, lavender, camelina, linseed, privet, lupine, lucerne, corn, almond, mirabelle, mango, poppy, evening primrose, olive, oilseed radish, oil palm, rocket, pecan, peach, plum, pistachio, cranberry, rapeseed, canola, rice, marigold, turnip rape, safflower, sage, sea buckthorn, black cumin, sesame, sesame leaf, mustard, sunflower, soy, soybean, walnut, grape, wheat, meadowfoam, wild rose and mixtures thereof. Fatty acids, or methyl esters/triglycerides thereof, such as palmitoleic acid, oleic acid, elaidinic acid, petroselinic acid, linoleic acid, linolenic acid, stearic acid, myristic acid, and gadoleic acid or mixtures thereof, can also be employed in the present invention.

Preferably, the linear or branched alkyl or linear alkenyl hydrocarbon group R proceeds from fatty acids derived from rapeseed oil, sunflower oil, soybean oil, more preferably from sunflower oil and rapeseed oil, even more preferably from high oleic sunflower oil (i.e. sunflower oil having a minimum 80 wt% oleic acid) and high oleic rapeseed oil (i.e. rapeseed oil having a minimum 80 wt% oleic acid).

Preferably, the linear alkyl or monounsaturated or polyunsaturated linear alkenyl hydrocarbon groups R are derived from vegetal oils, in particular from rapeseed oil, soybean oil and sunflower oil.

The degree of unsaturation of the vegetal oils can be determined by means of the iodine value. The iodine value (or iodine adsorption value or iodine number or iodine index, commonly abbreviated as IV) is the mass of iodine in grams that is consumed by 100 grams of a chemical substance or composition. Iodine value is commonly used to determine the amount of unsaturation in fats, oils and waxes. Thus, the iodine value is suitable to determine the degree of unsaturations of the vegetal oils of the present invention, said unsaturations being present in the acyl groups - COR defined herein. The higher the iodine value, the more unsaturations are present in the vegetal oils.

In an embodiment of the present invention, the linear alkyl or monosunsaturated or polyunsaturated linear alkenyl hydrocarbon groups R are derived from vegetal oils with a iodine value in the range from 75 to 200, preferably from 75 to 145, more preferably in the range from 75 to 110. The iodine value can be determined according to ISO 3961: Animal and vegetable fats and oils: Determination of iodine value (based in LIPSA material specification).

In an embodiment of the present invention, R is selected from a linear alkyl and monounsaturated or polyunsaturated linear alkenyl containing 14 to 20 carbon atoms; preferably selected from a linear alkyl containing 14 to 20 carbon atoms and linear alkenyl group containing 14 to 20 carbon atoms and from 1 to 3 double bonds; preferably, the alkyl or alkenyl group contains from 16 to 18 carbon atoms and the alkenyl group has from 1 to 3 double bonds.

In the compositions of the present invention, at least 50% by weight of the acyl groups -CO-R comprised in components (i), (ii) and (iii) of the composition are a -CO-$R_1$ group. In a preferred embodiment, at least 60% by weight of the acyl groups -CO-R comprised in components (i), (ii) and (iii) of the composition are a -CO-$R_1$ group, preferably at least 70% by weight, more preferably at least 80% by weight, even more preferably at least 85% by weight. As explained above, this group of formula $R_1$ is a monounsaturated or polyunsaturated linear alkenyl group having 18 carbon atoms, preferably it is a linear alkenyl group having 18 carbon atoms and from 1 to 3 double bonds; preferably it is a linear alkenyl group having 18 carbon atoms and 1 double bond (i.e. monounsaturated linear alkenyl group having 18 carbon atoms also named C18:1), a linear alkenyl group having 18 carbon atoms and 2 double bonds (i.e. also named C18:2) and a linear alkenyl group having 18 carbon atoms and 3 double bonds (i.e. also named C18:3); more preferably it is a linear alkenyl group having 18 carbon atoms and 1 double bond (i.e. a monounsaturated linear alkenyl group having 18 carbon atoms also named C18:1) and a linear alkenyl group having 18 carbon atoms and 2 double bonds (i.e. also named C18:2) ; even more preferably a linear alkenyl group having 18 carbon atoms and 1 double bond (i.e.

a monounsaturated linear alkenyl group having 18 carbon atoms also named C18:1). The wt% (or % by weight) refers to the amount by weight of acyl groups $COR_1$ with respect to the total amount of acyl groups in the composition, i.e. considering only those acyl groups in the composition having from 6 to 22 carbon atoms.

In an embodiment of the present invention, at least 60% by weight of the acyl groups -CO-R comprised in components (i), (ii) and (iii) of the composition are a $-CO-R_1$ group, wherein the $R_1$ is a linear alkenyl group having 18 carbon atoms and 1 double bond (i.e. a monounsaturated linear alkenyl group having 18 carbon atoms also named C18:1), preferably at least 70% by weight, more preferably at least 80% by weight; and/or at least 2% by weight of the acyl groups -CO-R comprised in components (i), (ii) and (iii) of the composition are a $-CO-R_1$ group, wherein the $R_1$ is a linear alkenyl group having 18 carbon atoms and 2 double bonds (i.e. also named C18:2), preferably from 2% to 25% by weight.

In another embodiment of the composition of the present invention, at least one R group in the composition is a group of formula $R_4$ that is a saturated linear alkyl group having 18 carbon atoms. The presence of at least one R group in the composition being a group of formula $R_4$ that is a saturated linear alkyl group having 18 carbon atoms means that at least one of components (i) to (iv) has at least one R group that is $R_4$. In the compositions of the present invention, up to 20% by weight of the acyl groups -CO-R comprised in components (i), (ii) and (iii) of the composition are a $-CO-R_4$ group, preferably up to 15% by weight, more preferably up to 10% by weight. The wt% (or percentage by weight) refers to the amount by weight of acyl groups $COR_4$ with respect to the total amount of acyl groups in the composition, i.e. considering only those acyl groups in the composition having from 6 to 22 carbon atoms.

In a particular embodiment of the compositions of the present invention, at least one R group in the composition is a group of formula $R_2$ that is a linear alkyl group or a monounsaturated or polyunsaturated linear alkenyl group having from 6 to 16 carbon atoms, preferably a linear alkyl having from 12 to 16 carbon atoms, more preferably a linear alkyl having 16 carbon atoms. The presence of at least one R group in the composition being a group of formula $R_2$ that is a linear alkyl group or a monounsaturated or polyunsaturated linear alkenyl group having from 6 to 16 carbon atoms means that at least one of components (i) to (iv) has at least one R group that is $R_2$. Preferably, no more than 20% by weight of the acyl groups -CO-R comprised in components (i), (ii) and (iii) of the composition are a $-CO-R_2$ group, more preferably no more than 15% by weight, even more preferably no more than 10% by weight, even more preferably no more than 7.5% by weight. The wt% refers to the amount by weight of acyl groups $COR_2$ with respect to the total amount of acyl groups in the composition, i.e. considering only those acyl groups in the composition having from 6 to 22 carbon atoms. In a preferred embodiment, no more than 7.5% by weight of the acyl groups -CO-R comprised in components (i), (ii) and (iii) of the composition are a $-CO-R_2$ group.

The percentages by weight of the acyl groups -CO-R wherein R is a $R_1$, $R_2$ and/or $R_4$ group in the compositions of the invention may be determined by Gas Chromatography, according to ISO 12966.

The degree of unsaturation of the compositions (i.e. the total degree of unsaturation due to the presence of components in the composition having unsaturations, such as the acyl groups -COR present in components (i), (ii) and (iii) described herein) can be determined using the iodine value. The iodine value (or iodine adsorption value or iodine number or iodine index, commonly abbreviated as IV) is the mass of iodine in grams that is consumed by 100 grams of a chemical substance or composition. Iodine value are commonly used to determine the amount of unsaturation in fats, oils and waxes. Thus, the iodine value is suitable to determine the degree of unsaturations of the compositions of the present invention, said unsaturations being present in the acyl groups - COR defined herein. The higher the iodine value, the more unsaturations are present in the compositions.

In a particular embodiment of the invention, the iodine value ("IV") of the compositions is in the range from 3 to 150, preferably from 5 to 120, more preferably in the range from 7 to 85, even more preferably in the range from 10 to 50. The iodine value can be determined according to ISO 3961: Animal and vegetable fats and oils: Determination of iodine value (based in LIPSA material specification).

In the compositions of the present invention, the ratio by weight of components (i)/(ii)/(iii) is 5-90 / 10-50 / up to 65, preferably 15-85 / 15-50 / up to 45, more preferably 30-85 / 15-50 / up to 25. The weight of each component (i), (ii) and (iii) is selected within the ranges previously defined so that the total weight to (i)+(ii)+(iii) is 100.

In an embodiment of the invention, the ratio by weight of components (i)/(ii)/(iii) is 5-89 / 10-50 / 1 to 65, preferably 15-84 / 15-50 / 1 to 45, more preferably 30-84 / 15-50 / 1 to 25. The weight of each component (i), (ii) and (iii) is selected within the ranges previously defined so that the total weight to (i)+(ii)+(iii) is 100.

In the compositions of the present invention, the ratio by weight of the sum of components (i)+(ii)+(iii) to component (iv), that is, [(i)+(ii)+(iii)]/(iv) is from 1:3 to 1:0.002, preferably from 1:2 to 1: 0.01, more preferably from 1:1.5 to 1:0.05.

In a particular embodiment, in the composition of the invention at least 80% by weight, preferably 85% by weight of the acyl groups -CO-R comprised in components (i), (ii) and (iii) of the composition are a $-CO-R_1$ group which is a monounsaturated or polyunsaturated linear alkenyl group having 18 carbon atoms.

In a particular embodiment, in the composition of the invention at least 80% by weight of the acyl groups -CO-R comprised in components (i), (ii) and (iii) of the composition are a $-CO-R_1$ group which is a monounsaturated linear

alkenyl group having 18 carbon atoms (i.e. a C18:1).

In a particular embodiment, in the composition of the invention at least 2% by weight, preferably from 2% to 25% by weight of the acyl groups -CO-R comprised in components (i), (ii) and (iii) of the composition are a -CO-$R_1$ group which is a linear alkenyl group having 18 carbon atoms and two double bonds (i.e. a C18:2).

The above wt% or percentage by weights refer to the amount by weight of acyl groups $COR_1$ as defined in each particular embodiment above with respect to the total amount of acyl groups in the composition, i.e. considering only those acyl groups in the composition having from 6 to 22 carbon atoms.

In a particular embodiment, the ratio by weight of components (i)/(ii)/ (iii) in the compositions of the invention is 30-85 / 15-50 / up to 25. The weight of each component (i), (ii) and (iii) is selected within the ranges previously defined so that the total weight to (i)+(ii)+(iii) is 100.

In another particular embodiment, in the composition of the invention the sum of m, n, l is from 5 to 10; at least 85% by weight of the acyl groups -CO-R comprised in components (i), (ii) and (iii) of the composition are a -CO-$R_1$ group which is a monounsaturated or polyunsaturated linear alkenyl group having 18 carbon atoms; at least 80% by weight of the acyl groups -CO-R comprised in components (i), (ii) and (iii) of the composition are a -CO-$R_1$ group which is a monounsaturated linear alkenyl group having 18 carbon atoms (i.e. a C18:1); at least 2% by weight of the acyl groups -CO-R comprised in components (i), (ii) and (iii) of the composition are a -CO-$R_1$ group which is a linear alkenyl group having 18 carbon atoms and two double bonds (i.e. a C18:2); the ratio by weight of components (i) / (ii)/ (iii) in the compositions of the invention is 30-85 / 15-50/ up to 25 (the weight of each component (i), (ii) and (iii) is selected within the ranges previously defined so that the total weight to (i)+(ii)+(iii) is 100).

In a preferred embodiment, the composition of the invention does not comprise any compound of formula (V)

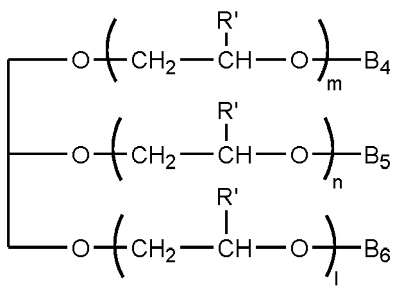

**(V)**

wherein R', m, n and l, and the sum of m, n and l, are as previously defined; and wherein $B_4$, $B_5$ and $B_6$ are independently selected from the group consisting of H and an acyl group represented by -CO-$R_3$, wherein $R_3$ represents independently a linear alkyl group or monounsaturated or polyunsaturated linear alkenyl group, containing 24 carbon atoms, preferably containing 22 to 24 carbon atoms.

The expression "does not comprise" means that no detectable amounts of compound (V) are present, preferably less than 1 wt%, more preferably less than 0.5 wt%, more preferably less than 0.1 wt%, even more preferably less than 0.01 wt%, the most preferred 0 wt%. Compound (V) can be detected by using conventional means in the art such as Gas Chromatography.

In another preferred embodiment, in the composition of the invention, at least 2% by weight, preferably from 2 to 25% by weight, of the acyl groups -CO-R comprised in components (i), (ii) and (iii) of the composition are a -CO-$R_1$ group wherein $R_1$ is a linear alkenyl group having 18 carbon atoms and two double bonds. The wt% refers to the amount by weight of acyl groups $COR_1$ with respect to the total amount of acyl groups in the composition, i.e. considering only those acyl groups in the composition having from 6 to 22 carbon atoms.

Preferably, the composition of the invention further comprises a mixture of

i') at least one component represented by formula (III), wherein one of the symbols $B_1$, $B_2$, $B_3$ independently represents an acyl group represented by -CO-R and the remaining ones represent H;

ii') at least one component represented by formula (III), wherein two of the symbols $B_1$, $B_2$, $B_3$ independently represent an acyl group represented by -CO-R and the remaining one represents H;

iii')at least one component represented by formula (III), wherein each one the symbols $B_1$, $B_2$, $B_3$ represent an acyl group represented by -CO-R;

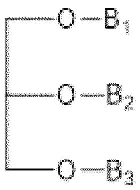

(III)

;

and

iv') at least one component represented by formula (IV);

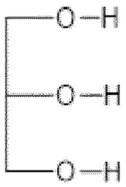

(IV)

wherein the acyl group represented by -CO-R , each R represents independently a linear alkyl group or mono-nounsaturated or polyunsaturated linear alkenyl group containing 6 to 22 carbon atoms, preferably 10 to 20 carbon atoms, more preferably 12 to 18 carbon atoms; provided that at least one R group is a group of formula $R_1$ that is a monounsaturated or polyunsaturated linear alkenyl group having 18 carbon atoms;
wherein at least 50% by weight of the acyl groups -CO-R comprised in components (i'), (ii') and (iii') of the composition are a-CO-$R_1$ group;
wherein the ratio by weight of components (i')/(ii')/(iii') is 5-90 / 10-50 / up to 65, preferably from 15-85 / 15-50 / up to 45, more preferably from 30-85 / 15-50 / up to 25; and wherein the ratio by weight of the sum of components (i')+(ii')+(iii') to component (iv'), is from 1:3 to 1:0.002, preferably from 1:2 to 1: 0.01, more preferably from 1:1.5 to 1:0.05.

Component (i') is a monoglyceride since only one of the symbols $B_1$, $B_2$, $B_3$ independently represents an acyl group represented by-CO-R and the remaining ones represent H. Component (i') comprises at least one monoglyceride of the compound of formula (III), that means that component (I') may comprise a single monoglyceride of the compound of formula (III) or it may comprise a mixture of two or more monoglycerides of the compound of formula (III).
Component (ii') is a diglyceride since two of the symbols $B_1$, $B_2$, $B_3$ independently represents an acyl group represented by -CO-R and the remaining one represents H. Component (ii') comprises at least one diglyceride of the compound of formula (III), that means that component (ii') may comprise a single diglyceride of the compound of formula (III) or it may comprise a mixture of two or more diglycerides of the compound of formula (III).
Component (iii') is a triglyceride since each one of the symbols $B_1$, $B_2$ and $B_3$ independently represent an acyl group represented by -CO-R. Component (iii') comprises at least one triglyceride of the compound of formula (III), that means that component (iii') may comprise a single triglyceride of the compound of formula (III) or it may comprise a mixture of two or more triglycerides of the compound of formula (III).
Component (iv) is glycerine.
In a particular embodiment of the present invention, in components (i'), (ii') and (iii'), R is selected from a linear alkyl and monounsaturated or polyunsaturated linear alkenyl containing 14 to 20 carbon atoms; preferably selected from a linear alkyl containing 14 to 20 carbon atoms and linear alkenyl group containing 14 to 20 carbon atoms and from 1 to 3 double bonds; preferably, the alkyl or alkenyl group contains from 14 to 18 carbon atoms and the alkenyl group has from 1 to 3 double bonds.
In another particular embodiment of the compositions of the present invention, at least 50% by weight of the acyl groups -COR comprised in components (i), (ii), (iii), (i'), (ii') and (iii') of the composition are a -CO-$R_1$ group. As explained above, this group of formula $R_1$ is a monounsaturated or polyunsaturated linear alkenyl group having 18 carbon atoms, preferably it is a linear alkenyl group having 18 carbon atoms and from 1 to 3 double bonds, more preferably it is a linear alkenyl group having 18 carbon atoms and 1 double bond (i.e. a monounsaturated linear alkenyl group having 18 carbon atoms also named C18:1) and a linear alkenyl group having 18 carbon atoms and 2 double bonds (i.e. also named C18:2); even more preferably a linear alkenyl group having 18 carbon atoms and 1 double bond (i.e. a monounsaturated linear alkenyl group having 18 carbon atoms also named C18:1). The wt% or percentage by weight refers to the amount by weight of acyl groups $COR_1$ with respect to the total amount of acyl groups in the composition, i.e. considering only those acyl groups in the composition having from 6 to 22 carbon atoms. In a preferred

embodiment, at least 60% by weight of the acyl groups -CO-R comprised in components (i), (ii), (iii), (i'), (ii') and (iii') of the composition are a -CO-$R_1$ group, preferably at least 70% by weight, more preferably at least 80% by weight, even more preferably at least 85% by weight.

In a particular embodiment, at least one R group in the mixture comprising components (i'), (ii'), (iii') and (iv') is a group of formula $R_2$ that is a linear alkyl group or a monounsaturated or polyunsaturated linear alkenyl group having from 6 to 16 carbon atoms, preferably a linear alkyl having 16 carbon atoms. Preferably, no more than 20% by weight of the acyl groups -CO-R comprised in components (i), (ii), (iii), (i'), (ii') and (iii') of the composition are a -CO-$R_2$ group, more preferably no more than 15% by weight, even more preferably no more than 10% by weight. The wt% or percentage by weight refers to the amount by weight of acyl groups $COR_2$ with respect to the total amount of acyl groups in the composition, i.e. considering only those acyl groups in the composition having from 6 to 22 carbon atoms. In a preferred embodiment, no more than 7.5% by weight of the acyl groups -CO-R comprised in components (i), (ii), (iii), (i'), (ii') and (iii') of the composition are a -CO-$R_2$ group.

In another embodiment, at least one R group in the mixture comprising components (i'), (ii'), (iii') and (iv') is a group of formula $R_4$ that is a saturated linear alkyl group having 18 carbon atoms. Preferably, up to 20% by weight of the acyl groups -CO-R comprised in components (i), (ii), (iii), (i'), (ii') and (iii') of the composition are a -CO-$R_4$ group, more preferably up to 15% by weight, even more preferably up to 10% by weight. The wt% or percentage by weight refers to the amount by weight of acyl groups $COR_2$ with respect to the total amount of acyl groups in the composition, i.e. considering only those acyl groups in the composition having from 6 to 22 carbon atoms.

In the compositions of the present invention, the ratio by weight of components (i')/(ii')/(iii') is 5-90 / 10-50 / up to 65, preferably 15-85 / 15-50 / up to 45, more preferably 30-85 / 15-50 / up to 25. The weight of each component (i'), (ii') and (iii') is selected within the ranges previously defined so that the total weight to (i')+(ii')+(iii') is 100.

In the compositions of the present invention, the ratio by weight of components (i+i')/(ii+ii')/(iii+iii') is 5-90 / 10-50 / up to 65, preferably 15-85 / 15-50 / up to 45, more preferably 30-85 / 15-50 / up to 25. The weight of (i+i'), (ii+ii') and (iii+iii') is selected within the ranges previously defined so that the total weight to (i+i')+(ii+ii')+(iii+iii') is 100.

In the compositions of the present invention, the ratio by weight of the sum of components (i')+(ii')+(iii') to component (iv'), that is, [(i')+(ii')+(iii')]/(iv') is from 1:3 to 1:0.002, preferably from 1:2 to 1: 0.01, more preferably from 1:1.5 to 1:0.05.

In of the compositions of the present invention, the ratio by weight of the sum of components (i)+(ii)+(iii)+(i')+(ii')+(iii') to the sum of components (iv)+(iv'), that is, [(i)+(ii)+(iii)+ (i')+(ii')+(iii')]/[(iv)+(iv')] is from 1:3 to 1:0.002, preferably from 1:2 to 1: 0.01, more preferably from 1:1.5 to 1:0.05.

**PROCESS TO OBTAIN THE MIXTURE OF MONO-, DI-, AND TRIGLYCERIDE AND GLYCERINE:**

The compositions of the present invention can be prepared as described in patent application EP1045021, i.e. by a first method that comprises the following steps:

a) reacting glycerine and a component of formula (IIIa) in an interesterification reaction, wherein R is as previously defined in the first aspect,

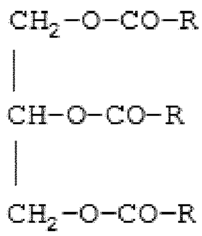

(IIIa)

and

b) subjecting the reaction mixture obtained in step a) to an alkoxylation process using an alkylene oxide having 2 or 3 carbon atoms in the presence of an alkaline catalyst.

The compound of formula (IIIa) is a compound of formula (III) wherein $B_1$, $B_2$ and $B_3$ are -COR groups, as in component (iii') previously described.

The interesterification reaction of step a) is governed by thermodynamics. Consequently, the molar proportion of components (i), (ii), (iii) and (iv) described above in the final product is determined by the proportion of starting materials,

glycerine and the component of formula (IIIa).

The component of formula (IIIa) includes vegetable oils and fats, as well as synthetic triglycerides, produced from esterification of fatty acids C14-C20 and glycerine.

Preferably, the reaction of step (a) takes place at a temperature from 150°C to 170°C. Preferably the reaction of step (a) is carried out at atmospheric pressure. Preferably, the reaction of step (a) is carried out during 2 to 3 hours. More preferably, the reaction of step (a) is carried out at a temperature from 150 °C to 170 °C, at atmospheric pressure and during 2 to 3 hours. The reaction of step (a) is preferably carried out in the absence of a solvent.

This reaction step provides a mixture of components (i')-(iv') as defined in the first aspect.

The subsequent alkoxylation reaction of step b) is a reaction which generally proceeds quantitatively, whereby the quantity of alkylene oxide used determines the degree of alkoxylation (i.e. the sum of m, n, and l).

Preferably, the alkylene oxide used in step b) is selected from the group consisting of ethylene oxide (when all R' in components (i)-(iv) are H), propylene oxide (when all in components (i)-(iv) R' are $CH_3$) and mixtures thereof (when R' in components (i)-(iv) is a mixture of H and $CH_3$). More preferably, the alkylene oxide is ethylene oxide.

Examples of alkaline catalysts are KOH, NaOH, $K_2CO_3$ and/or $NaOCH_3$.

Preferably, the reaction of step (b) takes place at a temperature from 150°C to 170°C. Preferably, the reaction of step (b) is carried out under pressure conditions of 2 to 3 $Kg/cm^2$. Preferably, the reaction of step (b) is carried out until all ethylene oxide is consumed. The consumption of ethylene oxide can be determined by common procedures in the art, such as Gas Chromatography. More preferably, the reaction of step (b) is carried out at a temperature from 150°C to 170°C, at pressure conditions of 2 to 3 $Kg/cm^2$, and until all ethylene oxide is consumed. The reaction of step (b) is preferably carried out in the absence of a solvent.

In alkoxylation step, the alkylene oxide reacts with any free moiety of the hydroxyl group, either from the mono-, diester and glycerine molecules obtained after step a), or from the ethoxylated glycerides that might be generated by further transesterification reaction during alkoxylation step. The proportion by weight of components (i), (ii), (iii) and (iv), consequently, is affected by alkoxylation. Despite the fact that the result of the reactions of steps a) and b) can be predicted by a person skilled in the art, it is possible to use model calculations to determine the correct proportion of the starting materials and thus obtain a proportion of predetermined and specific weights of components (i), (ii), (iii), and (iv) and a predetermined and specific degree of alkoxylation.

Furthermore, the compositions of the present invention can be produced by a second method which comprises the following steps: a') reacting glycerine with alkylene oxide having 2 or 3 carbon atoms in the presence of an alkaline catalyst; and b') the reacting the reaction mixture obtained in step a') with a component of formula (3)

R-COO-X (3) Formula (3)

wherein R is as defined in the first aspect and X represents a methyl group or H.

Preferably, the alkylene oxide used in step a') is selected from the group consisting of ethylene oxide (when all R' in components (i)-(iv) are H), propylene oxide (when all in components (i)-(iv) R' are $CH_3$) and mixtures thereof (when R' in components (i)-(iv) is a mixture of H and $CH_3$). More preferably, the alkylene oxide is ethylene oxide.

Preferably, the reaction of step (a') takes place at a temperature from 150°C to 170°C. Preferably, the reaction of step (a') is carried out at pressure conditions of 2 to 3 $Kg/cm^2$. Preferably, the reaction of step (a') is carried out until all ethylene oxide is consumed. Preferably, the reaction of step (a') is carried out at a temperature from 150°C to 170°C, at pressure conditions of 2 to 3 $Kg/cm^2$, and until all ethylene oxide is consumed. The reaction of step (a') is preferably carried out in the absence of a solvent.

The alkaline catalyst is as defined above for step b).

Preferably, the reaction of step (b') takes place at a temperature from 160°C to 180°C. Preferably, the reaction of step (b') is carried out at atmospheric pressure. Preferably, the reaction of step (b') is carried out until all component of formula (3) is consumed. Preferably, the reaction of step (b') is carried out a temperature from 160°C to 180°C, at atmospheric pressure, and until all component of formula (3) is consumed. The reaction of step (b') is preferably carried out in the absence of a solvent.

Examples of fatty acids of general formula (3) or of their methyl esters which are condensed (transesterified) with a mixture of glycerine with alkylene oxide with 2 or 3 carbon atoms are fatty acids C6-C22 from vegetable oils and fats. Preferably they are fatty acids derived from rapeseed oil, sunflower oil, palm stearin oil, more preferably from sunflower oil and rapeseed oil.

The degree of alkoxylation of the final product (i.e. the sum of m, n, and l) is determined by the quantity of alkylene oxide used in step a'). Next, step b') determines the molar proportion and the proportion by weight of components (i), (ii), (iii), and (iv). As before, the result of the reactions of steps a') and b') can be predicted by a person skilled in the art, so that it is possible to use model calculations to determine the correct proportion of starting materials and thus obtained a predetermined and specific proportion by weight of components (i), (ii), (iii) and (iv) and a predetermined and specific degree of alkoxylation.

Furthermore, the compositions of the present invention can be produced by a third method which consists of the following steps: a") reacting glycerine with alkylene oxide having 2 or 3 carbon atoms in the presence of an alkaline catalyst, and b") reacting the reaction mixture obtained in step a") is reacted with a component of formula (IIIa), as above described, wherein R is as defined in the first aspect.

Preferably, the alkylene oxide used in step a") is selected from the group consisting of ethylene oxide (when all R' in components (i)-(iv) are H), propylene oxide (when all in components (i)-(iv) R' are $CH_3$) and mixtures thereof (when R' in components (i)-(iv) is a mixture of H and $CH_3$). More preferably, the alkylene oxide is ethylene oxide.

The alkaline catalyst is as defined above for step b).

Preferably, the reaction of step (a'') takes place at a temperature from 150°C to 170°C. Preferably, the reaction of step (a") is carried out at pressure conditions of 2 to 3 $Kg/cm^2$. Preferably, the reaction of step (a") is carried out until all ethylene oxide is consumed. Preferably, the reaction of step (a'') is carried out at a temperature from 150°C to 170°C, at pressure conditions of 2 to 3 $Kg/cm^2$, and until all ethylene oxide is consumed. The reaction of step (b') is preferably carried out in the absence of a solvent.

Preferably, the reaction of step (b'') takes place at a temperature from 150°C to 170°C. Preferably, the reaction of step (b") is carried out at atmospheric pressure. Preferably, the reaction of step (b") is carried out for 4 to 8 hours. Preferably, the reaction of step (b'') is carried out at a temperature from 150°C to 170°C, at atmospheric pressure and for 4 to 8 hours. The reaction of step (b') is preferably carried out in the absence of a solvent.

This third process for obtaining the compositions of the invention is particularly suitable for obtaining compositions further comprising the mixture of components (I'), (ii'), (iii') and (iv').

In particular embodiments of the processes of the present invention, the alkylene oxide used in the process to obtain the compositions of the present invention has a synthetic or natural origin. In another embodiment of the invention, the alkylene oxide is ethylene oxide derived from ethanol having a vegetable origin.

## DETERGENT COMPOSITIONS:

### Detergent compositions

Another additional object of the present invention is the detergent compositions comprising the compositions of the invention as defined in the first aspect (mixture of alkoxylated mono-, di-, and triglycerides and glycerine and optionally non-alkoxylated mono-, di- and triglycerides) or a composition obtainable by the processes of the invention.

Another additional object of the present invention is the detergent compositions comprising the compositions of the invention as defined in the first aspect (mixture of alkoxylated mono-, di-, and triglycerides and glycerine and optionally non-alkoxylated mono-, di- and triglycerides), wherein the detergent composition comprises the composition of the invention as defined in the first aspect in an amount from 0.5 to 50% wt., preferably from 1 to 40% wt., more preferably from 1 to 35% wt., based on the total weight of the detergent composition.

The detergent compositions of the present invention may, additionally, contain one or more of the following additives, depending on the particular use intended for the detergent composition, without this being a limited list:

1. Anionic surfactants such as sodium alkyl ether sulfate, ammonium alkyl ether sulfate, triethanolamine alkyl ether sulfate, sodium alkyl sulfate, ammonium alkyl sulfate, triethanolamine alkyl sulfate, sodium alkyl sulfonate, sodium alkene sulfonate such as sodium alpha olefin sulfonate, sodium alkane sulfonate, sodium alkyl aryl sulfonate such as alkyl benzene sulfonate, sulfosuccinates, sarcosinates and sulfosuccinamates.
2. Fatty acids or soaps derived from natural or synthetic substances such as coconut, olive oil, soy and tallow fatty acids.
3. Ethoxylated alcohols, such as ethoxylated capryl alcohol, ethoxylated decyl alcohol, ethoxylated lauryl alcohol, ethoxylated myristyl alcohol, ethoxylated cetyl alcohol, ethoxylated palmitoleyl alcohol, ethoxylated stearyl alcohol, ethoxylated oleyl alcohol, ethoxylated behenyl alcohol.
4. Fatty acid esters derived from natural or synthetic substances such as glycol, ethylene glycol, diethylene glycol, propylene glycol, dipropylene glycol, sucrose, glucose or polyglycerine.
5. Ethoxylated esters of fatty acids such as hydroxy- type fatty acids.
6. Amphoteric surfactants such as alkyl amidopropyl betaine, alkyl betaine, alkyl amidopropyl sulfobetaine, alkyl sulfobetaine, cocoamphoacetates and cocoamphodiacetates.
7. Amine oxides such as dimethyl alkylamine oxides or alkyl amidopropylamine oxides.
8. Amides such as monoethanolamides, diethanolamides, ethoxylated amides or alkyl isopropanol amides.
9. Alkylglycosides and/or derivates thereof, such as sulfonated alkylglycosides, citrate alkylglycosides, tartrates alkylglycosides and carboxymethylated alkylglycosides.
10. Cationic surfactants such as alkyl benzyl dimethyl ammonium halides, alkyl trimethyl ammonium halides, quaternized ethoxylated amines, esterquats derived from triethanolamine, methyldiethanolamine, dimethylamine, propanediol and oligomers of said esterquats.

11. Perfumes, such as a neat perfume or a perfume microcapsule.
12. Additives to improve said formulations such as thickeners, pearling agents, opacifiers, antioxidants, preservatives or colouring agents.
13. Solvents such as propyleneglycol, ethanol, isopropanol.
14. Hydrotopes such as sodium cumene sulfonate, sodium xylene sulfonate.

In an embodiment of the invention, the detergent composition comprises the composition of the present invention as defined in the first aspect and water.

In an embodiment of the invention, the detergent composition comprises the composition of the present invention as defined in the first aspect and an anionic surfactant. Preferably, this composition also comprises water.

In another embodiment of the invention, the detergent composition comprises the composition of the present invention as defined in the first aspect and an alkylglycosides and/or derivatives thereof, preferably an alkylglycoside and/or a carboxymethylated alkylglycoside. Preferably, this composition also comprises water.

In a preferred embodiment, the detergent composition of the invention is selected from the group consisting of a laundry detergent composition (such as laundry detergents, laundry detergents with fabric care additional benefit, and liquid unit doses), a fabric care composition, a dishwashing composition (such as manual dishwashing composition), a hard surface detergent composition (such as glass; ceramics; metals, such as stainless steel and aluminum; and plastic materials such as SAN (styreneacrylonitrile), melamine or polypropylene, a skin cleansing composition and a hair cleansing composition.

In an embodiment of the invention, the detergent composition is a laundry detergent composition, wherein the laundry detergent composition comprises the composition of the invention as defined in the first aspect in an amount from 1 to 50% wt., preferably from 5 to 40% wt., based on the total weight of the detergent composition.

In another embodiment of the invention, the laundry detergent composition can be in the format of a standard detergent, in high concentrated format or in capsules format.

In a particular embodiment, the laundry detergent composition comprises:

a) from 1% wt. to 50% wt., preferably from 5 to 40% wt. of the composition according to the invention as defined in the first aspect,
b) from 1% wt. to 50% wt., preferably from 5% wt. to 40% wt. of anionic surfactant(s),
c) from 1% wt. to 40% wt., preferably from 1 to 25% of soap(s), and/or
d) from 0 to 90% wt. of water

wherein the wt.% are based on the total weight of the detergent composition.

In another embodiment of the invention, the detergent composition is a dishwashing composition, wherein the dishwashing composition comprises the composition of the invention as defined in the first aspect in an amount from 1% to 20% wt., preferably from 1% to 10% wt., based on the total weight of the detergent composition.

In another embodiment of the invention, the dishwashing composition can be in the format of a standard dishwashing composition, in high concentrated format or in concentrated dilutable dishwashing compositions.

In a particular embodiment, the dishwashing composition comprises:

a) from 1% wt. to 20% wt., preferably from 1% wt. to 10% wt. of the composition according to the invention as defined in the first aspect,
b) from 1% wt. to 60% wt., preferably from 10 to 50% wt. of anionic surfactant(s),
c) from 1% wt. to 30% wt., preferably from 3% wt. to 15% wt. of amphoteric surfactant(s), and/or
d) from 0 to 90% wt., preferably from 20% wt. to 80% wt. of water,

wherein the wt.% are based on the total weight of the detergent composition.

In another embodiment of the invention, the detergent composition is a hard surface cleaning composition, wherein the hard surface cleaning composition comprises the composition of the invention as defined in the first aspect in an amount from 0.5% to 20% wt., preferably from 2% to 15% wt., based on total weight of the detergent composition.

In a particular embodiment of the invention, the hard surface cleaning composition comprises:

a) from 0.5% wt. to 20% wt., preferably from 2% wt. to 15% wt. of the composition according to the invention as defined in the first aspect,
b) from 0 to 10% wt. of non-ionic surfactant(s),
c) from 0 to 10% wt. of anionic surfactant(s),
d) from 0 to 5% wt. of soap(s),

e) from 0 to 80% wt. of solvent(s), and/or
f) from 0 to 95% wt. of water,

wherein the wt.% are based on the total weight of the detergent composition.

PREPARATION OF DETERGENT COMPOSITION

The detergent composition of the present invention can be obtained following a conventional process of dispersing the composition defined in the first aspect or a composition obtainable by a process according to any of the second, third or fourth aspect, in water, preferably at a temperature in the range of 15 to 35 °C.

METHOD FOR CLEANSING:

According to a further aspect, the present invention provides a method for cleansing and/or caring of textiles or fabrics, dishware and hard surfaces, as well as skin and hair, comprising the step of applying a detergent composition as previously defined to textiles or fabrics, dishware and hard surfaces, as well as skin and hair, followed by a rinsing step with water.

USE OF THE DETERGENT COMPOSITION

The use of the compositions of the first aspect or a composition obtainable by a process as defined in the second, third or fourth aspect, as surfactant or co-surfactant in detergent compositions also forms part of the object of the invention, particularly in laundry detergent compositions, fabric care compositions, dishwashing compositions, hard surface detergent compositions or personal care compositions such as skin cleansing compositions, hair cleansing compositions and skin care compositions, more particularly in detergent compositions for washing fabrics, for manual dishwashing and for hard surface cleaning.

The composition of the present invention as defined in the first aspect is used, preferably, as surfactant or co-surfactant in detergent compositions wherein, preferably, the compositions of the present invention as defined in the first aspect are contained in quantities which range from 0.25 and 20% by weight, with respect to the total weight of the detergent composition, although more preferably between 0.5 and 8% by weight.

**COSMETIC CLEANSING COMPOSITIONS**

Another object of the present invention is a cosmetic cleansing composition comprising the compositions of the invention as defined in the first aspect or a composition obtainable by the process defined in the second, third or fourth aspects.

The composition of the present invention as defined in the first aspect or a composition obtainable by the process defined in the second, third or fourth aspects is used, preferably, as surfactant or co-surfactant in cosmetic cleansing compositions wherein, preferably, they are contained in quantities which range from 1 and 10% by weight, with respect to the total weight of the cosmetic cleansing composition, although more preferably between 1 and 5% by weight.

The cosmetic cleansing composition of the present invention may, additionally, contain one or more of the following additives, depending on the objective of the cosmetic cleansing composition compound, without this being a limited list: oil components, silicone, compounds, powders, further non-ionic surfactants, anionic surfactants, polymers, metal ion sequestering agents, UV protection factors, vitamins, antioxidants, antioxidant aids, perfume oils, germ inhibitors and the like as further auxiliaries and additives.

In a particular embodiment of the invention, the cosmetic cleansing composition comprises:

a) from 1% wt. to 10% wt., preferably from 1% wt. to 5% wt. of the composition according to the invention as defined in the first aspect,
b) from 1% wt. to 99% wt., preferably from 5% wt. to 99% wt. of anionic surfactant(s),
c) from 0 to 15 wt., preferably from 0.5% wt. to 15% wt. of amphoteric surfactant(s), and/or
d) from 0 to 95% wt. of water.

wherein the wt.% are based on the total weight of the cosmetic cleansing composition.

## PREPARATION OF THE COSMETIC CLEANSING COMPOSITION

The cosmetic cleansing composition of the present invention can be obtained following a conventional process of mixing the different components, well known by any skilled person. For example, the different components can be mixed up with water and then stirring to obtain a homogenous solution, preferably at a temperature in the range from 15 to 35ªC.

## METHOD FOR CLEANSING SKIN AND/OR HAIR

According to a further aspect, the present invention provides a method for cleansing the skin and/or hair comprising the steps of i) wetting or dampening the skin and/or hair; ii) applying a sufficient amount of a cosmetic cleansing composition comprising the composition according to the invention as defined in the first aspect or a composition obtainable by the process defined in the second, third or fourth aspects. The cosmetic cleansing composition can be applied directly on the skin and/or hair or by means of a cleansing instrument. Examples of cleansing instruments include, in a non-exclusive manner, brushes, sponges and meshes; and finally iii) rinsing the skin and/or hair with water.

## USE OF THE COSMETIC CLEANSING COMPOSITION

The use of the cosmetic cleansing composition comprising the compositions of the invention as defined in the first aspect or a composition obtainable by the process defined in the second, third or fourth aspects for the cleansing of skin and/or hair, particularly as shampoo, conditioning shampoo, gel cleanser, cream cleanser, foam cleanser, shower oil, shower cream, micellar cleansing water, non-rinse cleanser. Other possible applications of the cosmetic compositions comprising the compositions of the invention are related to the conditioning of hair and/or the care of skin.

## EXAMPLES:

The following examples are given in order to provide a person skilled in the art with a sufficiently clear and complete explanation of the present invention, but should not be considered as limiting of the essential aspects of its subject, as set out in the preceding portions of this description.

The first part of the Examples section refers to the preparation of the composition comprising a mixture of mono-, di-, and triglycerides and glycerine according to the invention as defined in the first aspect.

The second part (Examples 2 to 6) refers to the preparation of detergent and cleansing formulations according to the invention and the determination of performance evaluation of their properties.

### Example 1: Preparation of compositions comprising a mixture of mono-, di-, and triglycerides and glycerine

Preparation of composition 1 (according to the invention):

210.2 g (0.24 mol) of high oleic sunflower oil, 144.5 g (1.57 mol) of glycerine and 0.81 g of KOH 50% as catalyst are placed in a 2 Kg flask properly equipped. The system is purged several times with nitrogen, vacuum stripping is carried out until 100 °C, and heating is continued to 160 °C. After 2 h at 160 °C the reactor is pressurized to 2-3 Kg/cm$^2$ with ethylene oxide added until a total of 518.9 g (11.79 mol). After the final charge of ethylene oxide the reaction mixture is allowed to react for about one hour, cooled to 70 °C, bleached with 1.75 g of hydrogen peroxide 50% and neutralized with lactic acid 90% to pH=5-7.

Preparation of comparative composition 1 (comparative):

Comparative can be prepared in accordance with the procedure described in the examples of patent application EP-A-1045021.

The features of composition 1 according to the invention (E1) and comparative composition 1 (C1) are summarized in Table 1 below.

Table 1.- Mixtures of alkoxylated mono-, di-, and triglycerides and glycerine (percentages by weight)

| | Composition 1 (invention) E1 | Composition 1 (comparative) C1 |
|---|---|---|
| Alkyl chain (R ) source | High oleic Sunflower oil | Palm kernel oil |
| (i)/(ii)/(iii) | 81.2/17.6/1.2 | 76.5/21.6/1.8 |

(continued)

| | Composition 1 (invention) E1 | Composition 1 (comparative) C1 |
|---|---|---|
| ((i)+ (ii)+ (iii)) / (iv) | 1.0 | 1.3 |
| n+m+l for each component (i) to (iv) | 6.5 | 6.5 |
| % wt of C18:0 | 2.6 | 2.3 |
| % wt of C18:1 | 81.2 | 16.9 |
| % wt of C18:2 | 9.7 | 2.6 |
| % wt of C18:3 | 0.1 | <0.1 |
| % wt of C16:0 | 4.2 | 9.1 |
| % wt of C20 | <0.1 | <0.1 |
| % wt of C22 | <0.1 | <0.1 |
| % wt of C24 | <0.1 | <0.1 |
| Iodine value of composition | 20.2 | 4.9 |
| Iodine value of triglyceride raw material | 86 | 19.7 |

**Example 2: Liquid Detergent Compositions for Washing Clothes**

The liquid detergent compositions for washing clothes were prepared as indicated in Table 2.

Table 2.- Liquid Laundry Detergent Composition including the composition according to the invention (percentage by weight)

| Chemical description | % weight |
|---|---|
| Sodium laureth sulfate[1] | 8.5 |
| Example 1 product[2] | 6.0 |
| Coconut fatty acid | 2.3 |
| Sodium hydroxide | 0.2 |
| Sodium Citrate | 3.0 |
| Water | Up to 100 |
| | |
| pH as it is | 8.0 - 8.5 |
| [1] EMAL 270 at 70% active matter supplied by Kao Chemicals Europe<br>[2] As described in Example 1 | |

The compositions were prepared at room temperature, following a normal process for mixing the components, stirring after each addition, until they were completely homogenized.

Table 3 summarizes the appearance, the viscosity, and the evaluation of the detergency efficacy.

The appearance was evaluated visually at room temperature, designating the appearance in which the formula is transparent and homogeneous as "correct", and as "incorrect" if the composition shows cloudiness or phase separation.

The viscosity was measured at 20°C, using a Brookfield LV viscosimeter, the appropriate spindle type and speed (rpm) combination were chosen following the instructions of the Brookfield devices. If not indicated otherwise, the viscosity of liquid detergent compositions is measured with a spindle/speed combination of 2/6 at 20°C.

The detergency efficacy was established by determining the percentage of elimination of dirt present in standard fabric samples. The efficacy tests were carried out using a Miele Softtronic W5722 front-loading washing machine in the following conditions: synthetic/mix program, spin speed 800 rpm, washing water temperature 20°C, water hardness 20°HF (544 ppm $Ca^{2+}$ and 156 ppm $Mg^{2+}$), 2 kg pre-discharged cotton towel load and 55 grams of the detergent composition to be evaluated. Different types of standard dirt were used in each detergency test, placing 3 sets of dirty

fabrics specimens (5x5 cm dimensions) by wash. After the washing step, the fabric samples were left to air-dry at room temperature.

Detergency efficacy was determined by means of colorimetric measurements on the standard dirty fabric before and after the washing process. These measurements were taken using the colorimeter, for example Datacolor Spectro 700UV.

Detergency efficacy is expressed as % detergency, calculated from the following mathematical formula, in which the CIE L* (Lightness) parameter, which is from the colorimetric measurement, is involved.

$$\% \, detergency = \frac{L^*_{washed\ dirty\ fabric} - L^*_{unwashed\ dirty\ fabric}}{L^*_{unwashed\ "non\ dirty\ fabric"} - L^*_{unwashed\ dirty\ fabric}} \cdot 100$$

The percentage of detergency detailed in the table corresponds to the average value of all type of fabrics and the three specimens used for each type of fabric.

The type of dirt and fabrics used are Olive Oil / Carbon Black on cotton (E-101) and cotton-polyester (E-104), Pigment / Sebum on cotton (wfk 10D) and cotton-polyester (wfk 200), Lipstick on cotton (E-141) and Clay on cotton (wfk 10TE), for example from EMPA Swissatest Testmaterialien AG, and wfk-Testgewebe GmbH. The values shown in Table 3 demonstrate that the compositions according to the invention (E1) have a suitable formula appearance (in Table 3 described as "correct"), a better thickening ability showing higher viscosity values than comparison compositions (C1) and suitable cleaning power.

Table 3.- Appearance, viscosity and evaluation of the detergency efficacy of the liquid laundry detergent compositions.

| Composition of a liquid laundry detergent | Appearance | Viscosity (cP) spd2/6rpm | % Detergency efficacy (mean value) |
|---|---|---|---|
| C1 | Correct | 208 | 49.5 |
| E1 | Correct | 240 | 49.0 |

The softening performance and the anti-wrinkle effect are also evaluated for the liquid detergent compositions for washing clothes according to the invention:
Table 4 summarizes the softening performance and the anti-wrinkle effect.

The softening performance given by the use of the liquid laundry detergent compositions was determined by means of a sensorial test carried out by a panel of experts assessing pieces of terry cotton towel treated with the corresponding liquid laundry detergent compositions.

The terry cotton towels were treated using a Miele Softtronic W5722 front-loading washing machine in the following conditions: synthetic/mix program, spin speed 800 rpm, washing water temperature 20°C, water hardness 20°HF (544 ppm $Ca^{2+}$ and 156 ppm $Mg^{2+}$), 2 kg pre-discharged cotton towel load and 55 grams of the detergent to be evaluated. The treated cotton towels were finally spun dried and let dry by hanging, and left still for 24 hours under controlled atmospheric conditions (60% HR and 20°C).

The softening effect of the composition of the Example 1 was determined by comparison in pairs, by 12 panelists, against the reference (Comparative Composition 1). Results are indicated in Table 4. The comparative evaluation was made according to the following criteria:

+3: much softer than the reference
+2: softer than the reference
+1: slightly softer than the reference
0 as soft as the reference
-1: slightly harder than the reference
-2: harder than the reference
-3: much harder than the reference

The anti-wrinkle effect of the liquid laundry detergent compositions was determined by means of a visual assessment carried out by a panel of experts using cotton white shirts treated with the corresponding liquid laundry detergent compositions.

The cotton white shirts were submitted to the laundry process described above.

The anti-wrinkle effect of the composition of the Example 1 was determined by comparison in pairs, by 12 panelists, against the reference (Comparative composition 1). Results are indicated in Table 4. The comparative evaluation was made according to the criteria described above.

Table 4.- Softening performance and the anti-wrinkle effect of the Liquid Laundry Detergent Compositions on fabrics.

| | E1 |
|---|---|
| Softening performance | +1 |
| Anti-wrinkle effect | +1 |
| Compared to | C1 |

The values shown in Table 4 demonstrate that the compositions according to the invention (E1) have a better softening performance and anti-wrinkle effect than comparison composition (C1) .

**Example 3: Liquid Detergent Compositions for Washing Dishes**

The liquid detergent compositions for washing dishes were prepared as indicated in Table 5.

Table 5- Liquid hand-dishwashing composition including the composition of the invention (percentage by weight)

| Chemical description | % weight |
|---|---|
| Sodium laureth sulfate[1] | 14.4 |
| Cocamidopropylamine Oxide[2] | 7.2 |
| Example 1 product[3] | 2.6 |
| Citric acid | 0.5 |
| Water | Up to 100 |
| | |
| pH as it is | 7.5 |

[1]EMAL 270 at 70% active matter supplied by Kao Chemicals Europe [2]OXIDET L75 C at 33% active matter supplied by Kao Chemicals Europe [3]As described in Example 1

The compositions are prepared at room temperature, following a normal process for mixing the components, stirring after each addition, until they are completely homogenized.

Table 6 summarizes the appearance and the viscosity. The appearance and the viscosity are evaluated as described in Example 2.

The values shown in Table 6 demonstrate that the compositions according to the invention (Example 1) have a suitable formula appearance (in Table 6 described as "correct"), and a better thickening ability showing higher viscosity values than comparison composition (Comparison 1).

Table 6.- Appearance and viscosity of the liquid hand-dishwashing compositions.

| Composition of a liquid hand-dishwashing composition | Appearance | Viscosity (cP) spd2/6rpm |
|---|---|---|
| C1 | Correct | 845 |
| E1 | Correct | 1385 |

Table 7 summarizes the evaluation of the liquid hand-dishwashing compositions.

The evaluation of the different compositions was carried out from the determination of the foaming power in the presence of grease (olive oil), according to the following procedure.

The foam volume of an aqueous solution of the product to be tested was determined at a concentration of 1 g/L (of composition according to Table 5), at a water hardness of 20°HF (French degrees) and at a temperature of 40°C.

The measurements were carried out using a "SITA Foam Tester R-2000" stirrer (supplied by SITA Messtechnik GmbH), working at 1500 rpm, in stirring cycles of 10 seconds.

The foam volume was measured between each stirring cycle and the addition of each 50 μL of grease (olive oil).

When the foam volume (y-axis) is represented against the number of oil additions (x-axis), a parabolic-type

curve is obtained, with a maximum for the foam value.

To compare the behavior of the different products, the following curve parameter is taken into consideration:

- Number of theoretical plates: cut with x-axis (number of oil additions) which corresponds to a foam volume level of 50 mL in the decreasing part of the curve.

The values shown in Table 7 demonstrate that the compositions according to the invention (Example 1) have a higher number of theoretical plates than comparison composition (Comparison 1).

Table 7.- The Maximum foam volume and the number of theoretical plates of the liquid hand-dishwashing compositions.

| Composition of a liquid hand-dishwashing composition | Number of theoretical plates |
|---|---|
| C1 | 41 |
| E1 | 44 |

**Example 5: Shampoo Compositions**

The shampoo compositions were prepared as indicated in Table 8.

Table 11.- Shampoo compositions including the composition of the invention (percentage by weight)

| Chemical description | % weight |
|---|---|
| Sodium laureth sulfate[1] | 14.4 |
| Cocamidopropylamine Betaine[2] | 7.2 |
| Example 1 product[3] | 2.6 |
| Water | Up to 100 |
| | |
| pH as it is | 5.5 |

[1]EMAL 270 at 70% active matter supplied by Kao Chemicals Europe [2]BETADET HR at 30% active matter supplied by Kao Chemicals Europe [3]As described in Example 1

The compositions were prepared at room temperature, following a normal process for mixing the components, stirring after each addition, until they are completely homogenized.

Table 12 summarizes the evaluation of the shampoo compositions. The evaluation of the shampoo compositions was carried out by means of a sensorial test on hair.

The sensorial tests were carried out by a panel of 6 experts. A caucasian hair stress of approximately 10 g and 22 cm in length was washed in presence of 50 L of sebum with 0.5 mL of the evaluated shampoo composition. The tress was washed using tap water of approximately 19°f of water hardness.

The attributes considered are the foam speed, foam volume, the foam creaminess, the wet smoothness, and the detangling.

The sensory score is given with a reference sample (comparative composition C1) and the comparative evaluation was made according to the following criteria:

1: much lower performance than the reference sample
2: lower performance than the reference sample
3: reference sample
4: better performance than the reference sample
5: much better performance than the reference sample

The sensory score detailed in Table 12 corresponds to the comparative evaluation between the composition according to the invention (Example 1) and the comparison composition C1 as a reference sample.

The values shown in Table 12 demonstrate that the composition according to the invention (Example 1) present a better foam speed, foam volume, foam creaminess, wet smoothness, and detangling that the comparison composition C1, considered as a reference sample.

Table 12.- Sensory score the Shampoo composition.

| Sensory Score | E1 |
|---|---|
| Foam speed | 4 |
| Foam Volume | 4 |
| Foam Creaminess | 4 |
| Wet smoothness | 4 |
| Detangling | 4 |
| Compared to | C1 |

## Claims

**1.** A composition comprising:

(i) at least one component represented by formula (I), wherein one of the symbols $B_1$, $B_2$, $B_3$ independently represents an acyl group represented by -CO-R and the remaining ones represent H;
(ii) at least one component represented by formula (I), wherein two of the symbols $B_1$, $B_2$ and $B_3$ independently represent an acyl group represented by -CO-R and the remaining one represents H;
(iii) at least one component represented by formula (I), wherein each one of the symbols $B_1$, $B_2$ and $B_3$ independently represent an acyl group represented by -CO-R;

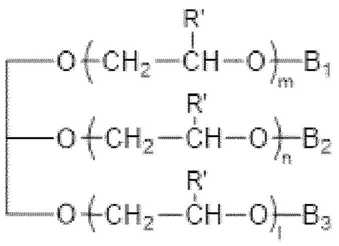

(I)

;

and
(iv) at least one component represented by formula (II);

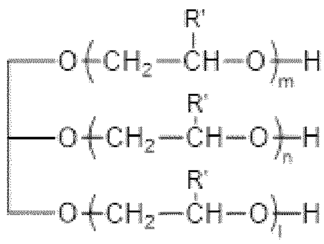

(II)

wherein each of m, n and l independently represents a number from 0 to 40,
the sum of m, n, l for each component (i)-(iv) is independently from 5 to 40, preferably from 5 to 30;
R' represents independently H or $CH_3$, preferably H;
wherein in the acyl groups represented by -CO-R, each R represents independently a linear alkyl group or a monounsaturated or polyunsaturated linear alkenyl group, containing 6 to 22 carbon atoms, preferably 10 to 20 carbon atoms, more preferably 12 to 18 carbon atoms, even more preferably from 14 to 18 carbon atoms; provided that at least one R group in the composition is a group of formula $R_1$ that is a monounsaturated or polyunsaturated linear alkenyl group having 18 carbon atoms;
wherein at least 50% by weight of the acyl groups -CO-R comprised in components (i), (ii) and (iii) of the composition are a -CO-$R_1$ group;

wherein the ratio by weight of components (i)/(ii)/(iii) is 5-90 / 10-50 / up to 65, preferably 15-85 / 15-50 / up to 45, more preferably 30-85 / 15-50 / up to 25; and
wherein the ratio by weight of the sum of components (i)+(ii)+(iii) to component (iv) is from 1:3 to 1:0.002, preferably from 1:2 to 1: 0.01, more preferably from 1:1.5 to 1:0.05.

**2.** A composition according to claim 1, wherein at least 60% by weight of the acyl groups -CO-R comprised in components (i), (ii) and (iii) of the composition are a -CO-$R_1$ group, preferably, at least 70% by weight, more preferably, at least 80% by weight.

**3.** A composition according to claim 1 or 2, wherein $R_1$ is a monounsaturated linear alkenyl group having 18 carbon atoms.

**4.** A composition according to any of claims 1 to 3, wherein in the acyl groups represented by -CO-R, at least one R group is a group of formula $R_2$ that is a linear alkyl group or a monounsaturated or polyunsaturated linear alkenyl group having from 6 to 16 carbon atoms.

**5.** A composition according to claim 4, wherein no more than 10% by weight of the acyl groups -CO-R comprised in components (i), (ii) and (iii) of the composition are a -CO-$R_2$ group.

**6.** A composition according to any of claims 1 to 5, wherein:

a) the composition does not comprise any compound of formula (V)

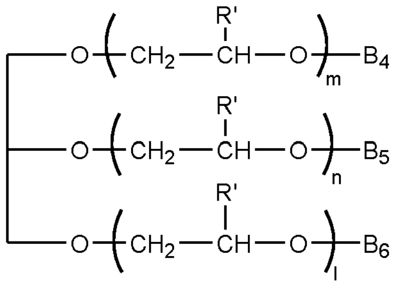

**(V)**

wherein R', m, n and l, and the sum of m, n and l, are as defined in claim 1; and
wherein $B_4$, $B_5$ and $B_6$ are independently selected from the group consisting of H and an acyl group represented by -CO-$R_3$, wherein $R_3$ represents independently a linear alkyl group or monounsaturated or polyunsaturated linear alkenyl group, containing 24 carbon atoms; and/or

b) at least 2% by weight, preferably from 2 to 25% by weight, of the acyl groups -CO-R comprised in components (i), (ii) and (iii) of the composition are a -CO-$R_1$ group wherein $R_1$ is a linear alkenyl group having 18 carbon atoms and two double bonds.

**7.** A composition according to claims 1 to 6, wherein the composition further comprises a mixture of

i') at least one component represented by formula (III), wherein one of the symbols $B_1$, $B_2$, $B_3$ independently represents an acyl group represented by -CO-R and the remaining ones represent H;
ii') at least one component represented by formula (III), wherein two of the symbols $B_1$, $B_2$, $B_3$ independently represent an acyl group represented by -CO-R and the remaining one represents H;
iii')at least one component represented by formula (III), wherein each one the symbols $B_1$, $B_2$, $B_3$ represent an acyl group represented by -CO-R;

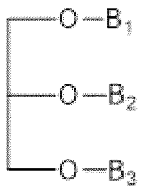

(III)

;

and

iv') at least one component represented by formula (IV);

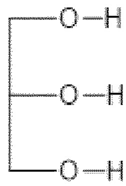

(IV)

wherein in the acyl group represented by -CO-R, each R is as defined in any one of the preceding claims; wherein at least 50% by weight of the acyl groups -CO-R comprised in components (i'), (ii') and (iii') of the composition are a - CO-$R_1$ group;
wherein the ratio by weight of components (i')/(ii')/(iii') is 5-90 /10-50 / up to 65, preferably 15-85 / 15-50 / up to 45, more preferably 30-85 / 15-50/ up to 25; and wherein the ratio by weight of the sum of components (i')+(ii')+(iii') to component (iv'), is from 1:3 to 1:0.002, preferably from 1:2 to 1: 0.01, more preferably from 1:1.5 to 1:0.05.

**8.** A process for producing the composition according to any one of claims 1 to 7, comprising the steps:

a) reacting glycerine with a component of formula (IIIa) in an interesterification reaction, wherein R is as defined in claims 1 to 7;

$CH_2$-O-CO-R
|
CH-O-CO-R
|
$CH_2$-O-CO-R

Formula (IIIa)

b) subjecting the reaction mixture obtained in step a) to an alkoxylation process using an alkylene oxide having 2 or 3 carbon atoms in the presence of an alkaline catalyst.

**9.** A process for producing the composition according to any one of claims 1 to 7, comprising the steps:

a') reacting glycerine with alkylene oxide of 2 or 3 carbon atoms in the presence of an alkaline catalyst;
b') reacting the reaction mixture obtained in step a') with a component of formula (3)

R-COOX (3)

wherein R is as defined in claims 1 to 7, and X represents a methyl group or H.

**10.** A process for producing the composition according to any one of claims 1 to 7, comprising the steps:

a") reacting glycerine with alkylene oxide of 2 or 3 carbon atoms in the presence of an alkaline catalyst;
b") reacting the reaction mixture obtained in step a") with a component of formula (IIIa) as defined in claim 8.

**11.** Detergent composition comprising a composition according to claim 1 to 7 or a composition obtainable by the process

as defined in claims 8 to 10, in an amount from 0.5 to 50% wt., preferably from 1 to 40% wt., more preferably from 1 to 35% wt., based on the total weight of the detergent composition.

**12.** Detergent composition according to claim 11 which is selected from the group consisting of a laundry detergent composition, a fabric care composition, a dishwashing composition, a hard surface detergent composition, a skin cleansing composition and a hair cleansing composition.

**13.** A process for producing the detergent composition according to claims 11 or 12 comprising the step of dispersing in water a composition as defined in claim 1 to 7 or obtainable by the process as defined in claims 8 to 10, preferably at a temperature in the range of 15 to 35 °C.

**14.** Use of the detergent compositions according to claims 11 or 12 for the cleansing and/or care of textiles or fabrics, dishware and hard surfaces as well as skin and hair.

**15.** A method for cleansing and/or caring of textiles or fabrics, dishware and hard surfaces, as well as skin and hair, comprising the step of applying a detergent composition as defined in claim 11 or 12 to textiles or fabrics, dishware and hard surfaces, as well as skin and hair.

**16.** Use of a composition according to any of claims 1 to 7 or a composition obtainable by the process as defined in claims 8 to 10 as surfactant or co-surfactant in a detergent composition, preferably wherein said detergent composition is selected from the group consisting of laundry detergent compositions, fabric care compositions, dishwashing compositions, hard surface detergent compositions or personal care compositions such as skin cleansing compositions, hair cleansing compositions and skin care compositions.

Europäisches Patentamt
European Patent Office
Office européen des brevets

# EUROPEAN SEARCH REPORT

Application Number
EP 23 38 2330

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2018/111746 A1 (HUNTSMAN PETROCHEMICAL LLC [US]) 21 June 2018 (2018-06-21)<br>* [0053], [0055], [0056], [0059], [0073], [0076], [0082], [0087], [00102], [00103], [00129]-[00132]; tables 3, 4, 5; Figures 3, 5 * | 1-7, 10-16<br>8,9, 11-16 | INV.<br>A61Q19/10<br>A61Q5/02<br>C11D1/825<br>C07C1/00<br>C07C67/03<br>C07C67/26<br>C07C69/58<br>C08G65/26<br>C11D11/00<br>C11D11/04<br>A61K8/39 |
| Y,D | EP 1 045 021 A1 (KAO CORP SA [ES]) 18 October 2000 (2000-10-18)<br>* claims 7 and 8; examples * | 8,9, 11-16 | |
| Y,D | EP 0 586 323 A1 (KAO CORP SA [ES]) 9 March 1994 (1994-03-09)<br>* claims 3 and 4; examples * | 8,9, 11-16 | |
| Y | EP 2 839 827 A1 (KAO CORP SA [ES]) 25 February 2015 (2015-02-25)<br>* claims 3 to 5 and examples * | 11-16 | |
| A | EP 1 479 754 A1 (KAO CHEMICALS GMBH [DE]) 24 November 2004 (2004-11-24)<br>* the whole document * | 1-16 | TECHNICAL FIELDS SEARCHED (IPC) |
| A | WO 03/011246 A1 (KAO CORP [JP]; CASTAN BARBERAN PILAR [ES]; ABE HIROSHI [ES]) 13 February 2003 (2003-02-13)<br>* the whole document * | 1-16 | C11D<br>A61K<br>C07C |

The present search report has been drawn up for all claims

1

| Place of search | Date of completion of the search | Examiner |
|---|---|---|
| The Hague | 14 September 2023 | Engelskirchen, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 38 2330

This annex lists the patent family members relating to the patent documents cited in the above-mentioned European search report. The members are as contained in the European Patent Office EDP file on
The European Patent Office is in no way liable for these particulars which are merely given for the purpose of information.

14-09-2023

| Patent document cited in search report | Publication date | Patent family member(s) | Publication date |
|---|---|---|---|
| WO 2018111746 A1 | 21-06-2018 | US 2020094207 A1 | 26-03-2020 |
| | | WO 2018111746 A1 | 21-06-2018 |
| EP 1045021 A1 | 18-10-2000 | AT 257171 T | 15-01-2004 |
| | | DE 69913934 T2 | 04-11-2004 |
| | | DK 1045021 T3 | 05-04-2004 |
| | | EP 1045021 A1 | 18-10-2000 |
| | | ES 2213939 T3 | 01-09-2004 |
| | | PT 1045021 E | 31-05-2004 |
| | | US 6265373 B1 | 24-07-2001 |
| | | US RE38639 E | 26-10-2004 |
| EP 0586323 A1 | 09-03-1994 | AT E136579 T1 | 15-04-1996 |
| | | DE 69302151 T2 | 05-12-1996 |
| | | EP 0586323 A1 | 09-03-1994 |
| | | ES 2088254 T3 | 01-08-1996 |
| EP 2839827 A1 | 25-02-2015 | BR 112014025808 B1 | 03-11-2020 |
| | | EP 2839827 A1 | 25-02-2015 |
| | | ES 2425998 A1 | 18-10-2013 |
| | | ES 2644305 T3 | 28-11-2017 |
| | | PL 2839827 T3 | 30-11-2017 |
| | | US 2015118328 A1 | 30-04-2015 |
| | | WO 2013156647 A1 | 24-10-2013 |
| EP 1479754 A1 | 24-11-2004 | EP 1479754 A1 | 24-11-2004 |
| | | EP 1629074 A1 | 01-03-2006 |
| | | EP 1675934 A1 | 05-07-2006 |
| | | WO 2004104150 A1 | 02-12-2004 |
| | | WO 2004104151 A1 | 02-12-2004 |
| WO 03011246 A1 | 13-02-2003 | AU 2002233332 A1 | 17-02-2003 |
| | | CN 1536985 A | 13-10-2004 |
| | | DE 60215662 T2 | 30-08-2007 |
| | | EP 1411892 A1 | 28-04-2004 |
| | | ES 2185497 A1 | 16-04-2003 |
| | | ES 2273993 T3 | 16-05-2007 |
| | | JP 3977328 B2 | 19-09-2007 |
| | | JP 2005501048 A | 13-01-2005 |
| | | US 2004214740 A1 | 28-10-2004 |
| | | WO 03011246 A1 | 13-02-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0586323 A
- EP 1045021 A
- EP 2029711 A
- EP 1106675 A